# EUROPEAN PATENT SPECIFICATION

(11) **EP 2 765 838 B1**
(45) Date of publication and mention of the grant of the patent: **11.07.2018**
(21) Application number: 14153957.7
(22) Date of filing: 05.02.2014
(51) Int. Cl.: H05H 1/46

(54) **System and method for controlling a hybrid polarized/non-polarized plasma beam**
System und Verfahren zur Kontrolle eines hybriden polarisierten/nichtpolarisierten Plasmastrahls
Système et procédé pour la commande d'un faisceau de plasma hybride polarisé/non polarisé

(30) Priority: 12.02.2013 US 201361763859 P; 20.11.2013 US 201314085339
(43) Date of publication of application: 13.08.2014
(73) Proprietor: Covidien LP, Mansfield, MA 02048 (US)
(72) Inventor: Friedrichs, Daniel, Aurora, CO 80012 (US); Sartor, Joe D., Longmont, CO 80504-7326 (US)
(74) Representative: Soames, Candida Jane

(56) References cited:
- WO-A2-2005/107857
- WO-A2-2011/055368
- JP-A- H11 290 335
- US-A1- 2001 018 587
- US-A1- 2005 101 947
- US-A1- 2006 041 257

## Description

### CROSS REFERENCE TO RELATED APPLICATION

The present application claims the benefit of and priority to U.S. Provisional Application Serial No. 61/763,859, filed on February 12, 2013.

### BACKGROUND

### Technical Field

The present disclosure relates to plasma devices and processes for surface processing and tissue treatment. More particularly, the disclosure relates to a system and method for generating and directing chemically reactive, plasma-generated species in a plasma device that can be selectively polarized or non-polarized.

### Background of Related Art

Electrical discharges in dense media, such as liquids and gases at or near atmospheric pressure, can, under appropriate conditions, result in plasma formation. Plasmas have the unique ability to create large amounts of chemical species, such as ions, radicals, electrons, excited-state (e.g., metastable) species, molecular fragments, photons, and the like. The plasma species may be generated in a variety of internal energy states or external kinetic energy distributions by tailoring plasma electron temperature and electron density. In addition, adjusting spatial, temporal and temperature properties of the plasma creates specific changes to the material being irradiated by the plasma species and associated photon fluxes. Plasmas are also capable of generating photons including energetic ultraviolet photons that have sufficient energy to initiate photochemical and photocatalytic reaction paths in biological and other materials that are irradiated by the plasma photons.

### SUMMARY

Plasmas have broad applicability and provide alternative solutions to industrial, scientific and medical needs, especially workpiece (e.g, tissue) surface treatment at any temperature range. Plasmas may be delivered to the workpiece, thereby affecting multiple changes in the properties of materials upon which the plasmas impinge. Plasmas have the unique ability to create large fluxes of radiation (e.g., ultraviolet), ions, photons, electrons and other excited-state (e.g., metastable) species which are suitable for performing material property changes with high spatial, material selectivity, and temporal control. Plasmas may also remove a distinct upper layer of a workpiece with little or no effect on a separate underlayer of the workpiece or it may be used to selectively remove a particular tissue from a mixed tissue region or selectively remove a tissue with minimal effect to adjacent organs of different tissue type.

The plasma species are capable of modifying the chemical nature of tissue surfaces by breaking chemical bonds, substituting or replacing surface-terminating species (e.g., surface functionalization) through volatilization, gasification or dissolution of surface materials (e.g., etching). With proper techniques, material choices and conditions, one can remove one type of tissue entirely without affecting a nearby different type of tissue. Controlling plasma conditions and parameters (including S-parameters, V, I, Θ, and the like) allows for the selection of a set of specific particles, which, in turn, allows for selection of chemical pathways for material removal or modification as well as selectivity of removal of desired tissue type.

WO 2011/055368 A2 discloses a compact medical device for tissue welding.

JPH11/290335 discloses an impedance selection system used in a plasma system to limit the high frequency current flowing through the body of a patient.

US2001/0018587 A1 discloses a high-frequency coagulation apparatus.

The present disclosure provides a plasma system including an electrosurgical generator; an ionizable media source; and a plasma instrument. The plasma instrument includes a housing having a lumen defined therein terminating in an opening at a distal end thereof, the lumen being in fluid communication with an ionizable media source and a pair of electrodes coupled to the electrosurgical generator. The plasma system also includes a return electrode pad configured to couple to a patient; and a polarization controller electrically coupled to the return electrode, the polarization controller configured to adjust conductive coupling of the return electrode pad to the electrosurgical generator.

According to one aspect of the present disclosure, the polarization controller includes a variable resistance.

According to one aspect of the present disclosure, the variable resistance includes a plurality of resistors coupled to a plurality of switching elements configured to switch the plurality of resistors into the variable resistance.

According to one aspect of the present disclosure, the variable resistance includes a variable potentiometer controllable by an electromechanical actuator.

According to one aspect of the present disclosure, the variable resistance includes a voltage-controlled resistance selected from the group consisting of a transistor, a PIN diode, and combinations thereof.

According to one aspect of the present disclosure, the electrosurgical generator and/or the plasma instrument include controls for adjusting resistance of the polarization controller.

According to one aspect of the present disclosure, while the variable resistance is fully activated the return electrode is decoupled from the electrosurgical generator and the plasma instrument outputs a non-polarized plasma.

According to one aspect of the present disclosure, while the variable resistance is fully deactivated the return electrode is coupled to the electrosurgical generator and the plasma instrument outputs a polarized plasma.

The present disclosure provides a plasma system including: an electrosurgical generator; an ionizable media source; and a plasma instrument. The plasma instrument includes a housing having a lumen defined therein terminating in an opening at a distal end thereof, the lumen being in fluid communication with an ionizable media source; and a pair of electrodes coupled to the electrosurgical generator. The plasma system further includes a return electrode pad configured to couple to a patient; and a polarization controller electrically coupled to the return electrode, the polarization controller including a variable resistance controllable by at least one of the electrosurgical generator or the plasma instrument.

According to one aspect of the present disclosure, the variable resistance includes a plurality of resistors coupled to a plurality of switching elements configured to switch the plurality of resistors into the variable resistance.

According to one aspect of the present disclosure, the variable resistance includes a variable potentiometer controllable by an electromechanical actuator.

According to one aspect of the present disclosure, the variable resistance includes a voltage-controlled resistance selected from the group consisting of a transistor, a PIN diode, and combinations thereof.

According to one aspect of the present disclosure, while the variable resistance is fully activated the return electrode is decoupled from the electrosurgical generator and the plasma instrument outputs a non-polarized plasma.

According to one aspect of the present disclosure, while the variable resistance is fully deactivated the return electrode is coupled to the electrosurgical generator and the plasma instrument outputs a polarized plasma.

According to one aspect of the present disclosure, the plasma instrument includes a slide switch configured to control the variable resistance.

According to one aspect of the present disclosure, the electrosurgical generator includes a touchscreen displaying a scale representative of a degree of polarization of the polarization controller.

The present disclosure provides a method including: supplying ionizable media to a plasma instrument; igniting the ionizable media at the plasma instrument by energizing a pair of electrodes disposed within the plasma instrument to form a plasma effluent; and adjusting variable resistance of a polarization controller coupled to a return electrode pad to control a degree of polarization of the plasma effluent.

According to one aspect of the present disclosure, the adjusting of the variable resistance includes sliding a slidable switch disposed on the plasma instrument.

According to one aspect of the present disclosure, the plasma instrument is coupled to an electrosurgical generator.

According to one aspect of the present disclosure, the adjusting of the variable resistance includes inputting a desired degree of polarization using a polarization scale displayed on a screen of the electrosurgical generator.

Any method aspects of the present disclosure may be implemented by control signals or instructions and/or may relate exclusively to control of the method aspects without extending to a step or steps of treating tissue.

In any of the system aspects of the present disclosure, the system is configured to establish an electric field between the return electrode, which is coupled to the patient, e.g. disposed on the skin of the patient, and one or both of the first and second electrodes of sufficient intensity to ionize the ionizable media, thereby forming polarized plasma.

In any one of the system aspects of the present disclosure, the system is configured to establish an electric field between the first and second electrodes to generate a non-polarized plasma within the instrument which is deliverable to the patient as the ionizable media is pushed out of the instrument.

In any of the system aspects of the present disclosure, the system is operable in a polarized and a non-polarized state and/or in a hybrid state.

In any of the system aspects of the present disclosure, the first and second electrodes are operable in a bipolar manner and conductive coupling of the return electrode is variable by the polarization controller to vary a degree of polarization of a plasma produced by the system.

In any of the system aspects of the present disclosure, the instrument is for issuing a plasma plume from the opening.

In any of the system aspects of the present disclosure, the first and second electrodes are arranged for capacitive coupling with the ionizable media.

In any of the system aspects of the present disclosure, energy is applicable through the first and second electrodes (optically arranged as bipolar electrodes) to form a plasma plume exiting through the opening.

In any of the system aspects of the present disclosure, the first and second electrodes are connected to the generator to allow the instrument to operate in a non-polarized manner.

In any of the system aspects of the present disclosure, the polarization controller is adjustable between a non-polarized state in which the return electrode is not coupled to the generator so that the instrument operates in a non-polarized manner with the first and second electrodes being coupled to the generator and energized, and a polarized state in which the return electrode pad is fully coupled to the generator allowing for the operation of the system in a polarized manner and/or the polarization controller is configurable in a hybrid state in which the return pad is partly coupled to the generator allowing for the operation of the system in a hybrid manner intermediate such polarized and non-polarized states. In an embodiment, the hybrid state is variable by changing the degree of partial coupling of the return electrode to vary the degree of polarization.

In any of the system aspects of the present disclosure, the first and second electrodes are arranged along the lumen. The present invention is defined by a plasma system according to claim 1, and by a method of controlling a degree of polarisation of a plasma effluent, the method not extending to the treatment of tissues.

Preferred embodiments of the present invention are defined in the appended dependent claims.

### BRIEF DESCRIPTION OF THE DRAWINGS

The accompanying drawings, which are incorporated in and constitute a part of this specification, illustrate exemplary embodiments of the disclosure and, together with a general description of the disclosure given above, and the detailed description of the embodiments given below, serve to explain the principles of the disclosure, wherein:
Fig. 1 is a perspective diagram of a plasma system according to the present disclosure;
Fig. 2 is a front view of one embodiment of an electrosurgical generator according to the present disclosure;
Fig. 3 is a schematic, block diagram of the embodiment of an electrosurgical generator of Fig. 2 according to the present disclosure;
Fig. 4 is a cross-sectional, side view of a plasma instrument of Fig. 1 according to the present disclosure; and
Fig. 5 is a schematic, block diagram of another embodiment of the plasma system of Fig. 1 according to the present disclosure.

### DETAILED DESCRIPTION

Plasmas may be generated using electrical energy that is delivered as either direct current (DC) electricity or alternating current (AC) electricity at frequencies from about 0.1 hertz (Hz) to about 100 gigahertz (GHz), including radio frequency ("RF", from about 0.1 MHz to about 100 MHz) and microwave ("MW", from about 0.1 GHz to about 100 GHz) bands, using appropriate generators, electrodes, and antennas. Choice of excitation frequency, the workpiece, as well as the electrical circuits that are used to deliver electrical energy to the workpiece affect many properties and requirements of the plasma. The performance of the plasma chemical generation, the delivery system and the design of the electrical excitation circuitry are interrelated -- as the choices of operating voltage, frequency and current levels (as well as phase) effect the electron temperature and electron density. Further, choices of electrical excitation and plasma device hardware also determine how a given plasma system responds dynamically to the introduction of new ingredients to the host plasma gas or liquid media.

Plasma beams may be used to coagulate, cauterize, or otherwise treat tissue through direct application of a high-energy plasma. In particular, kinetic energy transfer from the plasma to the tissue causes healing, and thus, affects thermal coagulation of bleeding tissue. Plasma beam coagulation utilizes a handheld electrosurgical instrument having one or more electrodes energizable by an electrosurgical generator, which outputs a high-intensity electric field suitable for forming plasma using ionizable media (e.g., inert gas).

Plasma beam coagulation systems may be polarized or non-polarized. As used herein, the term "polarized" refers to plasma systems that include a return electrode disposed outside the instrument, that is coupled to a patient (e.g., outside the treatment site). During operation of polarized plasma, the instrument including one or more active electrodes comes into close proximity with the patient, the electric field intensity becomes sufficient to ionize the gas thereby forming plasma. Plasma provides a conductive path to the patient and without being limited by any particular theory, it is believed that the clinical effect is primarily effected by resistance heating of the patient tissue as the electrosurgical current passes through the patient and to the return electrode.

As used herein, the term "non-polarized" refers to plasma systems that include a handheld electrosurgical instrument having both an active and a return electrode. The system does not include a separate return electrode coupled to the patient, thus isolating the patient from the electrosurgical generator. Electrosurgical energy is provided by the generator and forms an electric field between the electrodes contained within the instrument. In this configuration plasma is generated within the instrument and is delivered to the patient as gas is pushed out of the instrument. Without being bound by any particular theory, it is believed that primary clinical effect in non-polarized system is due to the transfer of kinetic and thermal energy of the plasma.

Polarized plasma systems produce faster coagulation that non-polarized systems. However, speed and degree of coagulation is difficult to control using conventional electrosurgical generators, since specialized circuitry is required that can vary plasma intensity without extinguishing the plasma. Further, polarized electric fields produced between the instrument and tissue are attracted and/or deflected by the plasma beam, making it difficult to aim the beam. Non-polarized systems avoid the aiming difficulty of polarized systems, but are slower in producing desired tissue effects. Furthermore, such systems also rely on specialized direct current generators, which have no utility in other electrosurgical modalities.

The present disclosure provides for a hybrid polarization plasma system that can be operated in polarized, non-polarized and hybrid manner to overcome the drawbacks of polarized and non-polarized systems. The system includes an electrosurgical generator and an ionizable media source. The system further includes a plasma instrument having two or more electrodes (e.g., bipolar) coupled to the generator and the ionizable media source and a return electrode in contact with a patient that is also coupled to the generator via a polarization controller having variable resistance. The system includes controls disposed at the generator and/or the instrument for adjusting the resistance of the polarization controller to adjust the degree of polarization of the plasma generated by the instrument. Thus, using a bipolar plasma surgical instrument and varying the extent to which the patient is electrically-coupled to the generator via the return electrode, allows for varying the degree of polarization of the plasma beam (e.g., from purely polarized to purely non-polarized) and therebetween.

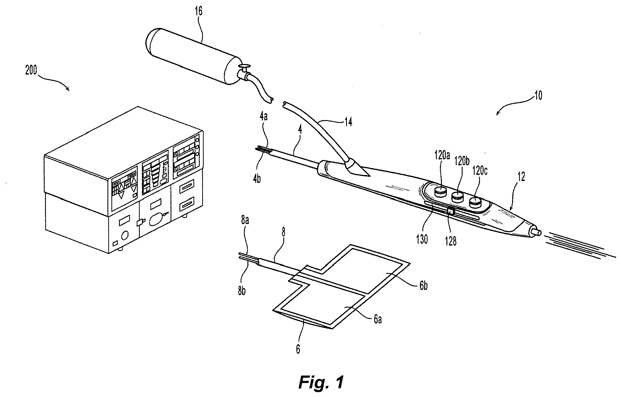

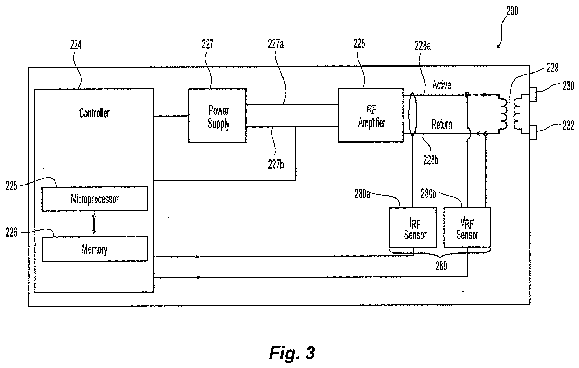

Referring initially to Fig. 1, a plasma system 10 is disclosed. The system 10 includes a plasma instrument 12 that is coupled to a generator 200, an ionizable media source 16 which may also include an optional precursor source (not shown). Generator 200 includes any suitable components for delivering power to the plasma instrument 12. More particularly, the generator 200 may be any radio frequency generator or other suitable power source capable of producing power to ignite the ionizable media to generate plasma. In embodiments, electrosurgical energy is supplied to the instrument 12 by the generator 200 via an instrument cable 4. The cable 4 includes a supply lead 4a connecting the instrument 12 to an active terminal 230 (Fig. 3) of the generator 200 and a return lead connecting the instrument 12 to a return terminal 232 (Fig. 3) of the generator 200. The plasma instrument 12 may be utilized as an electrosurgical pencil for application of plasma to tissue and the Generator 200 may be an electrosurgical generator that is adapted to supply the instrument 12 with electrical power at a frequency from about 100 kHz to about 4 MHz, in embodiments the frequency may range from about 200 kHz to about 3 MHz, in further embodiments the frequency may range from about 300 kHz to about 1 MHz.

The system 10 also includes one or more return electrode pads 6 that, in use, are disposed on a patient to minimize the chances of tissue damage by maximizing the overall contact area with the patient. The return electrode pad 6 may include two or more split electrodes 6a, 6b. The generator 200 may be configured to measure impedance between the split electrodes 6a, 6b to monitor tissue-to-patient contact to ensure that sufficient contact exists between the electrode pad 6 and the patient. The energy is returned to the generator 200 through the return electrode pad 6 via one or more return leads 8a, 8b, housed within a return pad cable 8 at the return terminal 232 (Fig. 3) of the generator 200. In particular, each of the return leads 8a, 8b is connected to one or more split electrodes 6a, 6b of the return electrode pad 6.

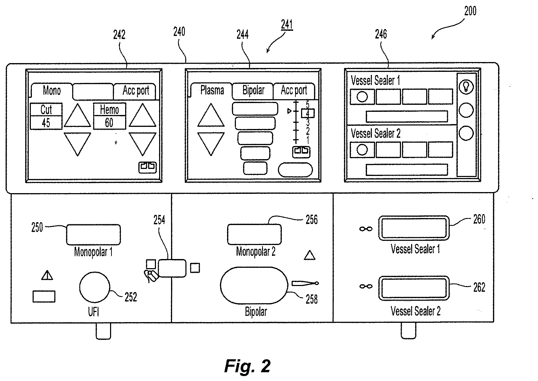

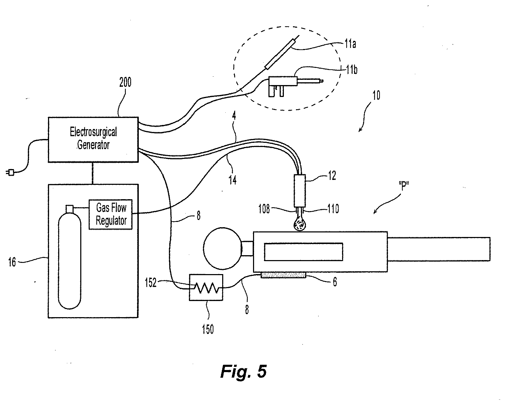

With reference to Fig. 2, a front face 240 of the generator 200 is shown. The generator 200 may be any suitable type (e.g., electrosurgical, microwave, etc.) and may include a plurality of connectors 250-262 to accommodate various types of electrosurgical instruments (e.g., electrosurgical forceps, electrosurgical pencils, ablation probes, etc.) in addition to the plasma instrument 12 as shown in Fig. 5.

The generator 200 includes a user interface 241 having one or more display screens 242, 244, 246 for providing the user with variety of output information (e.g., intensity settings, treatment complete indicators, etc.). Each of the screens 242, 244, 246 is associated with corresponding connector 250-262. The generator 200 includes suitable input controls (e.g., buttons, activators, switches, touch screen, etc.) for controlling the generator 200. The display screens 242, 244, 246 are also configured as touch screens that display a corresponding menu for the electrosurgical instruments (e.g., plasma instrument 12, etc.). The user then adjusts inputs by simply touching corresponding menu options.

Screen 242 controls monopolar output and the devices connected to the connectors 250 and 252. Connector 250 is configured to couple to a monopolar electrosurgical instrument (e.g., electrosurgical pencil) and connector 252 is configured to couple to a foot switch (not shown). The foot switch provides for additional inputs (e.g., replicating inputs of the generator 200). Screen 244 controls monopolar, plasma and bipolar output and the devices connected to the connectors 256 and 258. Connector 256 is configured to couple to other monopolar instruments. Connector 258 is configured to couple to plasma instrument 12.

Connector 254 may be used to connect to one or more return electrode pads 6. The return electrode pad 6 is coupled to the generator 200 via the return pad cable 8, which is coupled to the connector 254 via a plug (not shown). The return electrode pad 6 is coupled to a polarization controller 150, which is in turn coupled to the connector 254 (as shown in Fig. 5), which is described in further detail below. Screen 246 controls plasma procedures performed by the plasma instrument 12 that may be plugged into the connectors 260 and 262.

Fig. 3 shows a schematic block diagram of the generator 200 configured to output electrosurgical energy. The generator 200 includes a controller 224, a power supply 227, and a radio-frequency (RF) amplifier 228. The power supply 227 may be a high voltage, DC power supply connected to an AC source (e.g., line voltage) and provides high voltage, DC power to the RF amplifier 228 via leads 227a and 227b, which then converts high voltage, DC power into treatment energy (e.g., electrosurgical or microwave) and delivers the energy to the active terminal 230. The energy is returned thereto via the return terminal 232. The active and return terminals 230 and 232 and coupled to the RF amplifier 228 through an isolation transformer 229. The RF amplifier 228 is configured to operate in a plurality of modes, during which the generator 200 outputs corresponding waveforms having specific duty cycles, peak voltages, crest factors, etc. It is envisioned that in other embodiments, the generator 200 may be based on other types of suitable power supply topologies.

The controller 224 includes a processor 225 operably connected to a memory 226, which may include transitory type memory (e.g., RAM) and/or non-transitory type memory (e.g., flash media, disk media, etc.). The processor 225 includes an output port that is operably connected to the power supply 227 and/or RF amplifier 228 allowing the processor 225 to control the output of the generator 200 according to either open and/or closed control loop schemes. A closed loop control scheme is a feedback control loop, in which a plurality of sensors measure a variety of tissue and energy properties (e.g., tissue impedance, tissue temperature, output power, current and/or voltage, etc.), and provide feedback to the controller 224. The controller 224 then signals the power supply 227 and/or RF amplifier 228, which adjusts the DC and/or power supply, respectively. Those skilled in the art will appreciate that the processor 225 may be substituted by using any logic processor (e.g., control circuit) adapted to perform the calculations and/or set of instructions described herein including, but not limited to, field programmable gate array, digital signal processor, and combinations thereof.

The generator 200 according to the present disclosure includes a plurality of sensors 280, e.g., an RF current sensor 280a, and an RF voltage sensor 280b. Various components of the generator 200, namely, the RF amplifier 228, the RF current and voltage sensors 280a and 280b, may be disposed on a printed circuit board (PCB). The RF current sensor 280a is coupled to the active terminal 230 and provides measurements of the RF current supplied by the RF amplifier 228. In embodiments the RF current sensor 280a may be coupled to the return terminal 232. The RF voltage sensor 280b is coupled to the active and return terminals 230 and 232 provides measurements of the RF voltage supplied by the RF amplifier 228. In embodiments, the RF current and voltage sensors 280a and 280b may be coupled to active and return leads 228a and 228b, which interconnect the active and return terminals 230 and 232 to the RF amplifier 228, respectively.

The RF current and voltage sensors 280a and 280b provide the sensed RF voltage and current signals, respectively, to the controller 224, which then may adjust output of the power supply 227 and/or the RF amplifier 228 in response to the sensed RF voltage and current signals. The controller 224 also receives input signals from the input controls of the generator 200 and/or the plasma instrument 12. The controller 224 utilizes the input signals to adjust the power output of the generator 200 and/or performs other control functions thereon.

With reference once again to Fig. 1, the system 10 provides a flow of plasma through the instrument 12 to a workpiece (e.g., tissue). Plasma feedstocks, which include ionizable media and optional precursor feedstocks, are supplied by the ionizable media source 16 to the plasma instrument 12. During operation, the ionizable media and/or the precursor feedstock are provided to the plasma instrument 12 where the plasma feedstocks are ignited to form plasma effluent containing ions, radicals, photons from the specific excited species and metastables that carry internal energy to drive desired chemical reactions in the workpiece or at the surface thereof. The feedstocks may be mixed upstream from the ignition point or midstream thereof (e.g., at the ignition point) of the plasma effluent.

The ionizable media source 16 may include a storage tank, a pump, and/or flow meter (not explicitly shown). The ionizable media may be a liquid or a gas such as argon, helium, neon, krypton, xenon, radon, carbon dioxide, nitrogen, hydrogen, oxygen, etc. and their mixtures, and the like. These and other gases may be initially in a liquid form that is gasified during application. The precursor feedstock may be either in solid, gaseous or liquid form and may be mixed with the ionizable media in any state, such as solid, liquid (e.g., particulates or droplets), gas, and the combination thereof.

With continued reference to Fig. 1, the ionizable media source 16 may be coupled to the plasma instrument 12 via tubing 14. The tubing 14 may be fed from multiple sources of ionizible media and/or precursor feedstocks, which may combined into unified tubing to deliver a mixture of the ionizable media and the precursor feedstock to the instrument 12 at a proximal end thereof. This allows for the plasma feedstocks, e.g., the precursor feedstock and the ionizable gas, to be delivered to the plasma instrument 12 simultaneously prior to ignition of the mixture therein.

In another embodiment, the ionizable media and precursor feedstocks may be supplied at separate connections, such that the mixing of the feedstocks occurs within the plasma instrument 12 upstream from the ignition point such that the plasma feedstocks are mixed proximally of the ignition point.

In a further embodiment, the plasma feedstocks may be mixed midstream, e.g., at the ignition point or downstream of the plasma effluent, directly into the plasma. It is also envisioned that the ionizable media may be supplied to the instrument 12 proximally of the ignition point, while the precursor feedstocks are mixed therewith at the ignition point. In a further illustrative embodiment, the ionizable media may be ignited in an unmixed state and the precursors may be mixed directly into the ignited plasma. Prior to mixing, the plasma feedstocks may be ignited individually. The plasma feedstock may be supplied at a predetermined pressure to create a flow of the medium through the instrument 12, which aids in the reaction of the plasma feedstocks and produces a plasma effluent. The plasma according to the present disclosure may be generated at or near atmospheric pressure under normal atmospheric conditions.

In one embodiment, the precursors may be any chemical species capable of forming reactive species such as ions, electrons, excited-state (e.g., metastable) species, molecular fragments (e.g., radicals) and the like, when ignited by electrical energy from the Generator 200 or when undergoing collisions with particles (electrons, photons, or other energy-bearing species of limited and selective chemical reactivity) formed from ionizable media 16. More specifically, the precursors may include various reactive functional groups, such as acyl halide, alcohol, aldehyde, alkane, alkene, amide, amine, butyl, carboxlic, cyanate, isocyanate, ester, ether, ethyl, halide, haloalkane, hydroxyl, ketone, methyl, nitrate, nitro, nitrile, nitrite, nitroso, peroxide, hydroperoxide, oxygen, hydrogen, nitrogen, and combination thereof. In embodiments, the precursor feedstocks may be water, halogenoalkanes, such as dichloromethane, tricholoromethane, carbon tetrachloride, difluoromethane, trifluoromethane, carbon tetrafluoride, and the like; peroxides, such as hydrogen peroxide, acetone peroxide, benzoyl peroxide, and the like; alcohols, such as methanol, ethanol, isopropanol, ethylene glycol, propylene glycol, alkalines such as NaOH, KOH, amines, alkyls, alkenes, and the like. Such precursor feedstocks may be applied in substantially pure, mixed, or soluble form.

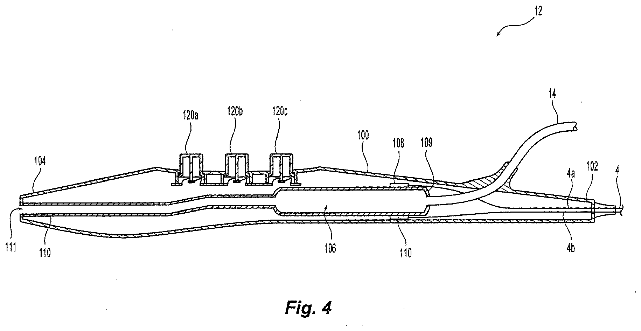

With reference to Figs. 1 and 4, the instrument 12 includes a handle housing 100 having a proximal end 102 and a distal end 104. The housing 100 also includes a lumen 106 defined therein having a proximal end 109 coupled to the gas tubing 14 from the ionizable media source 16 and a distal end 110 having an opening 111 terminating at the distal end 104 of the housing 100. The distal end 110 may have any suitable shape for tailoring the size and/or shape of the plasma plume generated by the instrument 12. In embodiments, the lumen 106 may include one or more surfaces for further shaping (e.g., narrowing) the plasma plume, such as a frustoconical portion 112, prior to exiting the instrument 12. The ionizable media source 16 may include various flow sensors and controllers (e.g., valves, mass flow controllers, etc.) to control the flow of ionizable media to the instrument 12. In particular, the lumen 106 is in gaseous and/or liquid communication with the ionizable media source 16 allowing for the flow of ionizable media and precursor feedstocks to flow through the lumen 106.

The instrument 12 includes two or more electrodes 108, 110 disposed within the lumen 106. The electrodes 108 and 110 may be formed from a conductive material and have any suitable shape for conducting electrical energy and igniting the ionizable media. The electrodes 108 and 110 may be shaped as rings, strips, needle, meshes, and the like. The electrodes 108 and 110 may be disposed outside the lumen 106 for capacitive coupling with the ionizable media. The ionizable media in conjunction with the optional precursor feedstocks is ignited by application of energy through the electrodes 108 and 110 to form a plasma plume exiting through the opening 111.

The electrodes 108 and 110 are coupled to conductors 4a, 4b, respectively, that extend through the housing 100 and are connected to the generator 200 via the cable 4. The cable 4 may include a plug (not shown) connecting the instrument 12 to the generator 200 at the connector 258. Each of the electrodes 108 and 110 is connected to the generator 200 and may therefore be energized by the generator 200 allowing the instrument 12 to operate in non-polarized manner as described in further detail below.

With reference to Figs. 1 and 4, the instrument 12 also includes one or more activation switches 120a-120c, each of which extends through top-half shell portion of housing 100. Each activation switch 120a-120c is operatively supported on a respective tactile element (e.g., a snap-dome switch) provided on a switch plate 124. Each activation switch 120a-120c controls the transmission of electrical energy supplied from generator 200 to the electrodes 108 and 110. The activation switches 120a-120c transmit control signals via a voltage divider network (VDN) or other circuit control means through control leads within the cable 4 to the generator 200. For the purposes herein, the term "voltage divider network" relates to any known form of resistive, capacitive or inductive switch closure (or the like) which determines the output voltage across a voltage source (e.g., one of two impedances) connected in series. A "voltage divider" as used herein relates to a number of resistors connected in series, which are provided with taps at certain points to make available a fixed or variable fraction of the applied voltage.

With reference to Fig. 1, the instrument 12 further includes a slide switch 128 slidingly supported on or within housing 100 in a guide channel 130 defined therein. The switch 128 may be configured to function as a slide potentiometer, sliding over and along VDN. The switch 128 has a first position at a proximal-most position (e.g., closest to cable 4) corresponding to 0 % or a relatively low polarization setting, a second position wherein the switch 128 is at a distal-most position (e.g., closest to opening 111) corresponding to 100 % or a relatively high polarization setting. The switch 128 may be disposed in a plurality of intermediate positions wherein the switch 128 is at positions between the distal-most position and the proximal-most position corresponding to various intermediate polarization settings. As can be appreciated, the polarization settings from the proximal end to the distal end may be reversed, e.g., high to low. Activation switches 120a-120c and the switch 128 are described in further detail in a commonly-owned U.S. Patent No. 7,879,033.

Fig. 5 shows the system 10 for applying plasma to a patient "P." The return electrode pad 6 is coupled to the patient. The return electrode pad 6 may be disposed underneath the patient "P" such that the patient "P" rests on top thereof. In embodiments, the return electrode pad 6 may be coupled to the patient "P" with conductive hydrogels and/or adhesives. As shown in Fig. 5, the system 10 may also include monopolar and bipolar surgical instruments 11a, 11b, respectively, which may be energized by the generator 200 to treat tissue.

The return electrode pad 6 is coupled to the polarization controller 150, which is in turn coupled to the connector 254 of the generator 200 via the cable 8. In embodiments, two or more return electrode pads 6 may be coupled to the patient "P." A splitter (not shown) may be used to couple multiple return electrode pads 6 to the generator 200 (e.g., at the connector 254). The splitter may be coupled to the polarization controller 150 prior to being connected to the generator 200. In further embodiments, multiple polarization controllers 150 may be utilized to accommodate a plurality of return electrode pads 6. In embodiments, the polarization controller 150 may be disposed within the generator 200 and be coupled to the input of the return electrode pad 6 at the generator 200 (e.g., connector 254).

The polarization controller 150 includes a variable resistance 152, which may be adjusted to control the conductive coupling of the return electrode pad 6 to the generator 200. Based on the conductivity of the return electrode pad 6, the instrument 12 may be operated in polarized, non-polarized, or hybrid manner. In particular, with the variable resistance 152 being fully activated such that the return electrode pad 6 is not coupled to the generator 200, the instrument 2 operates in a non-polarized manner, with the electrodes 108, 110 being only coupled to the generator 200 and therefore, being energized. With the variable resistance 152 being fully deactivated such that the return electrode pad 6 is fully-coupled to the generator 200. In this instance, the electrodes 108, 110 of the instrument 12 in combination with the return electrode pad 6 are connected to the generator 200 allowing for the operation of the system 10 in polarized manner.

Variable resistance 152 may include a plurality of resistors having a predetermined resistance that may be switched in and out of the circuit using switching elements (e.g., relays, transistors, field effect transistors, etc.). In embodiments, the variable resistance 152 may also include a variable potentiometer controllable by an electromechanical actuator. In further embodiments, the variable resistance 152 may include voltage-controlled resistances such as one or more transistors operated in its linear region or other semiconductor-based, electrically-controlled variable resistances, such as PIN diodes. The variable resistance 152 may be adjusted in fixed increments. In the present invention the variable resistance is infinitely variable (e.g., limited by electrical/physical limitations of its constituent components) or combinations thereof. Switching resistance in variable manner allows for fine-tuning the polarization of the system 10 to achieve a desired electrosurgical effect (e.g., maintaining constant current or constant voltage through the return electrode pad 6 or the instrument 12). Switching the resistance in a fixed manner (e.g., switching between fully-connected or fully-disconnected) variable resistance 152, allows for switching between non-polarized or polarized configurations, respectively.

Resistance of the polarization controller 150 may be controlled either through the generator 200 and/or the instrument 12. With reference to Fig. 2, the screen 244 may be a touchscreen that allows for control of the outputs the connectors 256 and 258 as well as the resistance of the polarization controller 150. In embodiments, the screen 244 may be replaced and/or supplemented by other controls (e.g., keyboard, buttons, etc.). The screen 244 includes input buttons for adjusting the degree of polarization. This may be accomplished by a variety of control schemes, shown on the screen 244 as graphical user interface elements, such as a slidable bar, predefined increment buttons, text and/or number inputs, and combinations thereof. The polarization settings may be displayed as a percentage or any other suitable scale for conveying the degree of polarization. The polarization settings are used by the generator 200 to adjust the resistance of the polarization controller 150, namely, the variable resistance 152 as described above to achieve a desired degree of polarization of the plasma outputted by the instrument 12.

The instrument 12 may also control various properties of the plasma beam. The activation switches 120a-120c may be used to activate the generator 200 and/or to control the flow of ionizable media from the ionizable media source 16. The slide switch 128 is configured to adjust the resistance of the polarization controller 150, namely, the variable resistance 152 as described above to achieve a desired degree of polarization of the plasma outputted by the instrument 12.

In embodiments, additional input devices may be used such as foot switches or handheld keyboards and/or remotes. The input devices (e.g., activation switches 120a-120c) may be two-stage switches where upon activation of the first stage, ionizable media and RF energy are supplied to the instrument 12 at a sufficient level to prime the active plasma field within the lumen 106 to initiate non-therapeutic ionization. This enables the user to visualize the target tissue relative to the non-therapeutic ionized gas plume. The generator 200 may include a feedback control loop to ensure the pre-ionization level is achieved and maintained at minimum needed RF power. In embodiments, a single wave spike may be generated to maintain sufficient ionized field without over heating the plasma instrument by minimizing RMS power delivered to pre-ionization field. In further embodiments, trace amounts of substantially non-electronegative compositions may be added to improve visibility of the ionized gas. Suitable tracer compositions include compounds such as sodium, neon, xenon, combinations thereof, and the like.

The closure of the second stage of the switch increases RF power to therapeutic levels and simultaneously increases conductivity through the return electrode pad 6 thereby initiating targeted therapeutic results. In particular, activation of the second stage would decrease the resistivity of the variable resistance 152 as described above.

The present disclosure provides for a plasma electrosurgical system with variable polarization, which allows for real-time adjustment of the plasma beam, thereby allowing for achieving specific surgical effects. The system also allows for used of standard electrosurgical generators (e.g., non-resonance matching generators operating in the radio frequency range at about 400 kHz) as a power source for exciting the plasma. Thus, a single electrosurgical generator may be used for generating plasma as well as operating with conventional electrosurgical instruments (e.g., monopolar, bipolar, etc.), thereby reducing the cost of operating room equipment.

Although the illustrative embodiments of the present disclosure have been described herein with reference to the accompanying drawings, it is to be understood that the disclosure is not limited to those precise embodiments, and that various other changes and modifications may be effected therein by one skilled in the art without departing from the scope or spirit of the disclosure. In particular, as discussed above this allows the tailoring of the relative populations of plasma species to meet needs for the specific process desired on the workpiece surface or in the volume of the reactive.

The present invention is defined and limited only by the appended claims.

## Claims

1. A plasma system (10) comprising:
an electrosurgical generator (200);
an ionizable media source (16);
a plasma instrument (12) comprising:
a housing (100) having a lumen (106) defined therein terminating in an opening (111) at a distal end thereof, the lumen being in fluid communication with the ionizable media source (16); and
a pair of electrodes (108, 110) coupled to the electrosurgical generator (200); and
a return electrode pad (6) configured to couple to a patient; and
a polarization controller (150) electrically coupled to the return electrode pad (6);
**characterised in that**
the plasma instrument (12) comprises a slide switch (128) and **in that**
the polarization controller (150) includes an infinitely variable resistance (152), and wherein at least one of the electro surgical generator (200) or the slide switch (128) of the plasma instrument (12) is configured to adjust said infinitely variable resistance (152).

2. The plasma system as claimed in claim 1 wherein the polarization controller (150) is configured to adjust the conductive coupling of the return electrode pad (6) to the electrosurgical generator (200) by adjusting the infinitely variable resistance (152).

3. The plasma system according to claim 1 or 2, wherein the infinite variable resistance (152) of the polarization controller (150) comprises a plurality of resistors coupled to a plurality of switching elements configured to switch the plurality of resistors into the infinitely variable resistance (152) of the polarization controller (150).

4. The plasma system according to claim 1, 2 or 3 wherein the infinite variable resistance (152) of the polarization controller (150) comprises a variable potentiometer controllable by an electromechanical actuator.

5. The plasma system according to any preceding claim, wherein the infinitely variable resistance (152) of the polarization controller (150) comprises a voltage-controlled resistance selected from the group consisting of a transistor, a PIN diode, and combinations thereof.

6. The plasma system according to any preceding claim, wherein the infinitely variable resistance (152) is configured to decouple the return electrode pad (6) from the electrosurgical generator (200) when fully activated, such that the plasma instrument (12) outputs a non-polarised plasma.

7. The plasma system according to any preceding claim, wherein the resistance is configured to couple the return electrode pad (6) to the electrosurgical generator (200) when fully deactivated, such that the plasma instrument (12) outputs a polarised plasma.

8. The plasma system according to any preceding claim, wherein the electrosurgical generator (200) includes a touchscreen (242;244;246) configured to display a scale representative of a degree of polarization of the polarization controller (150).

9. The plasma system according to any preceding claim, wherein the polarization controller (150) is configured to allow for varying the degree of polarization of plasma effluent formed by the instrument (12) from polarized to non-polarized and therebetween.

10. The plasma system according to any preceding claim, wherein the polarization controller (150) is adjustable between a non-polarized state in which the return electrode pad (6) is not coupled to the electrosurgical generator (200) so that the instrument (12) operates in a non-polarized manner with the first and second electrodes (108,110) being coupled to the electrosurgical generator (200) and energized, and a polarized state in which the return electrode pad (6) is fully coupled to the electrosurgical generator (200) allowing for the operation of the system in a polarized manner and the polarization controller (150) is configurable in a hybrid state in which the return electrode pad (6) is partly coupled to the electrosurgical generator (200) allowing for the operation of the system in a hybrid manner intermediate between polarized and non-polarized states, wherein the hybrid state is variable by changing the degree of partial coupling of the return electrode pad (6) to vary the degree of polarization through the infinitely variable (152) of the polarization controller (150).

11. A method of controlling a degree of polarization of a plasma effluent, the method not extending to treating tissues, the method comprising providing control instructions or signals configured to:
supply ionizable media to a plasma instrument (12);
ignite the ionizable media at the plasma instrument (12) by energizing a pair of electrodes (108,110) disposed within the plasma instrument (12) to form a plasma effluent; **characterised by** adjusting an infinitely variable (152) resistance of a polarization controller (150) coupled to a return electrode pad (6) to control a degree of polarization of the plasma effluent.

## Patentansprüche

1. Plasmasystem (10), umfassend:
einen elektrochirurgischen Generator (200);
eine ionisierbare Medienquelle (16);
ein Plasmainstrument (12), umfassend:
ein Gehäuse (100) mit einem darin definierten Lumen (106), welches in einer Öffnung (111) an einem distalen Ende davon endet, wobei das Lumen in Fluidverbindung mit der ionisierbaren Medienquelle (16) steht; und
ein Paar von Elektroden (108, 110), welches mit dem elektrochirurgischen Generator (200) verbunden ist; und
ein Rückführungselektrodenpad (6), welches konfiguriert ist, mit einem Patienten verbunden zu werden; und
einen Polarisierungskontroller (150), welcher mit dem Rückführungselektrodenpad (6) elektrisch verbunden ist;
**dadurch gekennzeichnet, dass** das Plasmainstrument (12) einen Schiebeschalter (128) umfasst, und dass der Polarisierungskontroller (150) einen unendlich variablen Widerstand (152) beinhaltet, und wobei zumindest einer von dem elektrochirurgischen Generator (200) oder dem Schiebeschalter (128) des Plasmainstruments (12) konfiguriert ist, den unendlich variablen Widerstand (152) einzustellen.

2. Plasmasystem nach Anspruch 1, wobei der Polarisierungskontroller (150) konfiguriert ist, die leitfähige Verbindung des Rückführungselektrodenpads (6) mit dem elektrochirurgischen Generator (200) mittels Einstellen des unendlich variablen Widerstands (152) einzustellen.

3. Plasmasystem nach Anspruch 1 oder 2, wobei der unendlich variable Widerstand (152) des Polarisierungskontrollers (150) eine Vielzahl von Widerständen umfasst, welche mit einer Vielzahl von Schaltelementen verbunden sind, welche konfiguriert sind, die Vielzahl von Widerständen in den unendlich variablen Widerstand (152) des Polarisierungskontrollers (150) zu schalten.

4. Plasmasystem nach Anspruch 1, 2, oder 3, wobei der unendlich variable Widerstand (152) des Polarisierungskontrollers (150) ein variables Potentiometer umfasst, welches mittels eines elektromechanischen Betätigers steuerbar ist.

5. Plasmasystem nach einem der vorstehenden Ansprüche, wobei der unendlich variable Widerstand (152) des Polarisierungskontrollers (150) einen spannungsgesteuerten Widerstand, ausgewählt aus der Gruppe, bestehend aus einem Transistor, einer PIN-Diode und Kombinationen davon, umfasst.

6. Plasmasystem nach einem der vorstehenden Ansprüche, wobei der unendlich variable Widerstand (152) konfiguriert ist, das Rückführungselektrodenpad (6) von dem elektrochirurgischen Generator (200), wenn vollständig aktiviert, zu trennen, sodass das Plasmainstrument (12) ein nicht polarisiertes Plasma ausgibt.

7. Plasmasystem nach einem der vorstehenden Ansprüche, wobei der Widerstand konfiguriert ist, das Rückführungselektrodenpad (6) mit dem elektrochirurgischen Generator (200), wenn vollständig deaktiviert, zu verbinden, sodass das Plasmainstrument (12) ein polarisiertes Plasma ausgibt.

8. Plasmasystem nach einem der vorstehenden Ansprüche, wobei der elektrochirurgische Generator (200) einen Touchscreen (242; 244; 246) beinhaltet, welcher konfiguriert ist, eine Skala zur Darstellung eines Grades der Polarisierung des Polarisierungskontrollers (150) anzuzeigen.

9. Plasmasystem nach einem der vorstehenden Ansprüche, wobei der Polarisierungskontroller (150) konfiguriert ist, eine Variation des Grades der Polarisierung von Plasmaabfluss, welcher von dem Instrument (12) gebildet wird, von polarisiert auf nicht polarisiert und dazwischen zu ermöglichen.

10. Plasmasystem nach einem der vorstehenden Ansprüche, wobei der Polarisierungskontroller (150) einstellbar ist zwischen einem nicht polarisierten Zustand, in welchem das Rückführungselektrodenpad (6) nicht mit dem elektrochirurgischen Generator (200) verbunden ist, sodass das Instrument (12) auf eine nicht polarisierte Weise arbeitet, wobei die ersten und zweiten Elektroden (108, 110) mit dem elektrochirurgischen Generator (200) verbunden und gespeist sind, und einem polarisierten Zustand, in welchem das Rückführungselektrodenpad (6) vollständig mit dem elektrochirurgischen Generator (200) verbunden ist und den Betrieb des Systems auf eine polarisierte Weise erlaubt, und der Polarisierungskontroller (150) in einem Hybridzustand konfigurierbar ist, in welchem das Rückführungselektrodenpad (6) teilweise mit dem elektrochirurgischen Generator (200) verbunden ist und den Betrieb des Systems in einem Hybridzustand in der Mitte zwischen polarisiertem und nicht polarisiertem Zustand erlaubt, wobei der Hybridzustand mittels Ändern des Grades der teilweisen Verbindung des Rückführungselektrodenpads (6) variabel ist, um den Grad der Polarisierung durch den unendlich variablen Widerstand (152) des Polarisierungskontrollers (150) zu variieren.

11. Verfahren zur Steuerung eines Grads der Polarisierung eines Plasmaabflusses, wobei sich das Verfahren nicht auf die Behandlung von Gewebe erstreckt, wobei das Verfahren das Bereitstellen von Steuerungsanweisungen oder Signalen umfasst, welche konfiguriert sind, um:
ionisierbare Medien an ein Plasmainstrument (12) zu liefern:
die ionisierbaren Medien in dem Plasmainstrument (12) durch Speisen eines Paares von Elektroden (108, 110), welche innerhalb des Plasmainstruments (12) angeordnet sind, zu zünden, um einen Plasmaabfluss zu bilden;
**gekennzeichnet durch** Einstellen eines unendlich variablen (152) Widerstands eines Polarisierungskontrollers (150), welcher mit einem Rückführungselektrodenpad (6) verbunden ist, um einen Grad der Polarisierung des Plasmaabflusses zu steuern.

## Revendications

1. Système à plasma (10) comprenant :
un générateur électrochirurgical (200) ;
une source de milieux pouvant être ionisés (16) ;
un instrument à plasma (12) comprenant :
un boîtier (100) ayant une lumière (106) définie en son sein se terminant dans une ouverture (111) au niveau d'une extrémité distale de celui-ci, la lumière étant en communication à fluide avec la source de milieux pouvant être ionisés (16) ; et
un couple d'électrodes (108, 110) couplées au générateur électrochirurgical (200) ;
et
une plage d'électrode de retour (6) configurée pour être couplée à un patient ; et
une unité de commande de polarisation (150) électriquement couplée à la plage d'électrode de retour (6) ;
**caractérisé en ce que**
l'instrument à plasma (12) comprend un commutateur à glissière (128) et **en ce que**
l'unité de commande de polarisation (150) inclut une résistance infiniment variable (152),
et dans lequel au moins un du générateur électrochirurgical (200) ou du commutateur à glissière (128) de l'instrument à plasma (12) est configuré pour ajuster ladite résistance infiniment variable (152).

2. Système à plasma selon la revendication 1, dans lequel l'unité de commande de polarisation (150) est configurée pour ajuster le couplage conducteur de la plage d'électrode de retour (6) au générateur électrochirurgical (200) en ajustant la résistance infiniment variable (152).

3. Système à plasma selon la revendication 1 ou 2, dans lequel la résistance variable infinie (152) de l'unité de commande de polarisation (150) comprend une pluralité d'éléments résistants couplés à une pluralité d'éléments de commutation configurés pour commuter la pluralité d'éléments résistants dans la résistance infiniment variable (152) de l'unité de commande de polarisation (150).

4. Système à plasma selon la revendication 1, 2 ou 3, dans lequel la résistance variable infinie (152) de l'unité de commande de polarisation (150) comprend un potentiomètre variable pouvant être commandé par un dispositif d'actionnement électromécanique.

5. Système à plasma selon l'une quelconque des revendications précédentes, dans lequel la résistance infiniment variable (152) de l'unité de commande de polarisation (150) comprend une résistance commandée en tension sélectionnée à partir du groupe constitué par un transistor, une diode PIN et des combinaisons de ceux-ci.

6. Système à plasma selon l'une quelconque des revendications précédentes, dans lequel la résistance infiniment variable (152) est configurée pour découpler la plage d'électrode de retour (6) du générateur électrochirurgical (200) quand entièrement activé, de sorte que l'instrument à plasma (12) sort un plasma non polarisé.

7. Système à plasma selon l'une quelconque des revendications précédentes, dans lequel la résistance est configurée pour coupler la plage d'électrode de retour (6) au générateur électrochirurgical (200) quand entièrement désactivé, de sorte que l'instrument à plasma (12) sort un plasma polarisé.

8. Système à plasma selon l'une quelconque des revendications précédentes, dans lequel le générateur électrochirurgical (200) inclut un écran tactile (242 ; 244 ; 246) configuré pour afficher une échelle représentative d'un degré de polarisation de l'unité de commande de polarisation (150).

9. Système à plasma selon l'une quelconque des revendications précédentes, dans lequel l'unité de commande de polarisation (150) est configurée pour permettre de faire varier le degré de polarisation d'effluent de plasma formé par l'instrument (12) de polarisé à non polarisé et entre ces derniers.

10. Système à plasma selon l'une quelconque des revendications précédentes, dans lequel l'unité de commande de polarisation (150) est réglable entre un état non polarisé dans lequel la plage d'électrode de retour (6) n'est pas couplée au générateur électrochirurgical (200) de sorte que l'instrument (12) fonctionne d'une façon non polarisée avec les première et seconde électrodes (108, 110) couplées au générateur électrochirurgical (200) et excitées, et un état polarisé dans lequel la plage d'électrode de retour (6) est entièrement couplée au générateur électrochirurgical (200) permettant le fonctionnement du système d'une façon polarisée et l'unité de commande de polarisation (150) peut être configurée dans un état hybride dans lequel la plage d'électrode de retour (6) est en partie couplée au générateur électrochirurgical (200) permettant le fonctionnement du système d'une façon hybride intermédiaire entre les états polarisé et non polarisé, dans lequel
l'état hybride est variable en changeant le degré de couplage partiel de la plage d'électrode de retour (6) pour faire varier le degré de polarisation par l'intermédiaire de l'infiniment variable (152) de l'unité de commande de polarisation (150).

11. Procédé de commande d'un degré de polarisation d'un effluent de plasma, le procédé ne s'étendant pas au traitement de tissus, le procédé comprenant la fourniture d'instructions ou de signaux de commande configurés pour :
fournir des milieux pouvant être ionisés à un instrument à plasma (12) ;
allumer les milieux pouvant être ionisés au niveau de l'instrument à plasma (12) en excitant un couple d'électrodes (108, 110) disposées à l'intérieur de l'instrument à plasma (12) pour former un effluent de plasma ; **caractérisé par**
l'ajustement d'une résistance infiniment variable (152) d'une unité de commande de polarisation (150) couplée à une plage d'électrode de retour (6) pour commander un degré de polarisation de l'effluent de plasma.
